# EUROPEAN PATENT SPECIFICATION

(11) **EP 2 780 335 B1**
(45) Date of publication and mention of the grant of the patent: **10.04.2019**
(21) Application number: 12823213.9
(22) Date of filing: 14.11.2012
(51) Int. Cl.: C07D 277/56

(54) **PROCESS FOR THE PREPARATION OF FEBUXOSTAT POLYMORPHS**
VERBESSERTES VERFAHREN ZUR HERSTELLUNG VON FEBUXOSTATPOLYMORPHEN
PROCÉDÉ DESTINÉ À LA PRÉPARATION DE POLYMORPHES DU FEBUXOSTAT

(30) Priority: 15.11.2011 IN 3909CH2011
(43) Date of publication of application: 24.09.2014
(73) Proprietor: Mylan Laboratories, Limited, Jubilee Hills, Hyderabad 500 034 (IN)
(72) Inventor: JETTI, Ramakoteswara, Rao, Hyderabad 500055, Andhra Pradesh (IN); BHOGALA, Balakrishna, Reddy, Hyderabad 500055, Andhra Pradesh (IN); BEERAVELLI, Satish, Hyderabad 500055, Andhra Pradesh (IN)
(74) Representative: FRKelly
(86) International application number: PCT/IN2012/000748
(87) International publication number: WO 2013/076738

(56) References cited:
- CN-A- 101 759 656
- CN-A- 101 817 801
- CN-B- 101 671 315

## Description

### FIELD OF THE INVENTION:

The present invention relates to a process for the preparation of Febuxostat Form K.

### BACKGROUND OF THE INVENTION:

Febuxostat, i.e. 2-(3-cyano-4-isobutyloxyphenyl)-4-methyl-5-thiazolecarboxylic acid of Formula-I is approved by USFDA for the treatment of hyperuricemia in patients with gout under the brand name of ULORIC. ULORIC is recommended at 40 mg or 80 mg once daily.

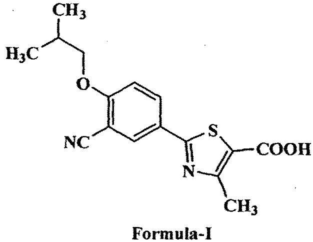

Febuxostat and its pharmaceutically acceptable salts were first disclosed in United States patent publication US 5,614,520. This patent also discloses process for the preparation of Febuxostat.

US 6225474 discloses crystalline Forms A, B, C, D, G and amorphous form of Febuxostat. This patent also discloses method of producing crystalline Forms A, B, C, D, G and amorphous Form of Febuxostat.

Crystalline Forms F1, F2, F10, R, R1, R2, R3, R4 and R5 of Febuxostat are disclosed US 20100317702, WO 2011107911 and WO 2011080651.

CN 101817801 discloses crystalline Form-K characterized by powder X-ray diffraction pattern having peaks at 5.65, 7.91, 11.52, 12.77, 14.29, 15.42, 16.75, 17.44, 18.14, 18.39, 20.47, 20.98, 22.23, 23.31, 23.81, 24.45, 25.89, 26.08, 28.92, 31.26 and 34.41±0.2 2Ø.

The present invention provides stable and industrially scalable crystalline form of Febuxostat and an improved process for the preparation of Febuxostat crystalline Form-K.

### OBJECT AND SUMMARY OF THE INVENTION:

An object of the present invention is to provide an improved process for the preparation of Febuxostat crystalline Form-K.

One aspect of the present invention provides an improved process for the preparation of crystalline Form-K of Febuxostat comprising the steps of:
a) dissolving Febuxostat in an ester solvent at 50 - 80 °C,
b) filtering the reaction mass,
c) maintaining the filtrate for 30-120 minutes at same temperature,
d) removing the solvent,
e) adding hydrocarbon solvent, and
f) isolating Febuxostat crystalline Form-K.

Preferably the ester solvent is ethyl acetate; and/or the hydrocarbon solvent is selected from cyclohexane or toluene.

In one aspect the invention provides an improved process for the preparation of crystalline Form-K of Febuxostat comprising the steps of:
a) dissolving Febuxostat in an ester solvent at 50 - 80 °C,
b) removing the solvent and
c) isolating Febuxostat crystalline Form-K.

Preferably the ester solvent is ethyl acetate.

In one apect the invention provides an improved process for the preparation of crystalline Form-K of Febuxostat comprising the steps of:
a. dissolving Febuxostat or an ethyl ester of Febuxostat in one or more organic solvents,
b. adding a base to the reaction mixture,
c. removing the solvent by distillation at 60-70°C,
d. cooling the reaction mass to 25-30°C, adding water and neutralizing with hydrochloric acid,
e. adding one or more ester solvents,
f. removing the ester solvent by distillation at 75-80°C under reduced pressure,
g. cooling the reaction mass to 25-30°C,
h. adding cyclohexane, and
i. isolating Febuxostat crystalline Form-K.

Preferably
i. the organic solvent is selected from ethanol, methanol, isopropanol, tetrahydrofuran or mixtures thereof; and/or
ii. the base is selected from alkali or alkali earth metal hydroxides; and/or
iii. the ester solvent is ethyl acetate.

### BRIEF DESCRIPTION OF THE DRAWINGS:

Further objects of the present invention together with additional features contributing thereto and advantages accruing there from will be apparent from the following description of preferred embodiments of the invention which are shown in the accompanying drawing figures wherein:
Figure 1 illustrates the powder X-ray diffraction pattern of Febuxostat Form-M₁.
Figure 2 illustrates DSC thermogram of Febuxostat Form-M₁.

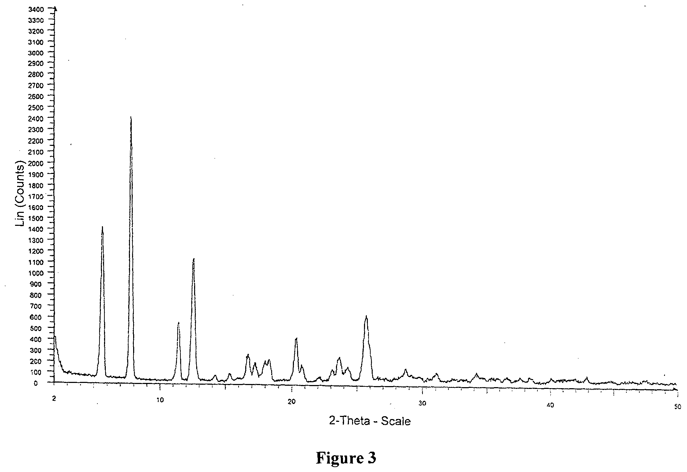

Figure 3 illustrates the powder X-ray diffraction pattern of Febuxostat Form-K

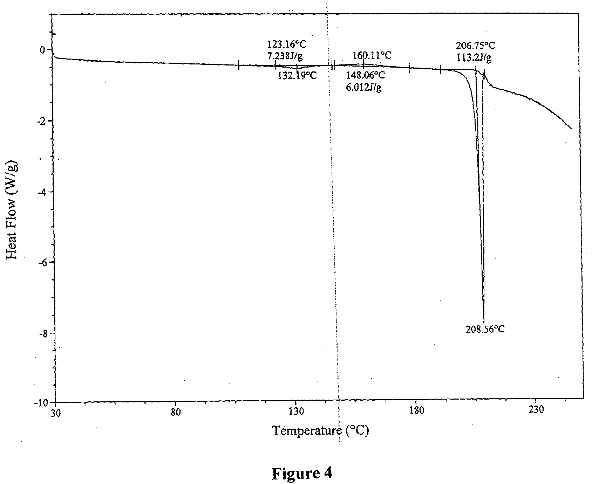

Figure 4 illustrates DSC thermogram of Febuxostat Form-K.

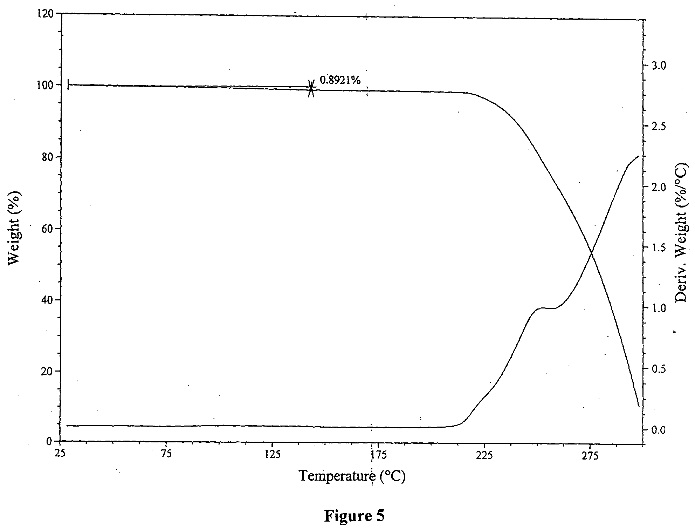

Figure 5 illustrates TGA thermogram of Febuxostat Form-K.
Figure 6 illustrates thermal ellipsoid plot of Febuxostat Form-K with atomic numbering scheme.
Figure 7 illustrates powder X-ray diffraction patterns of Febuxostat Form-K (Experimental and Simulated pattern).

### DETAILED DESCRIPTION OF THE INVENTION:

The present invention relates to the preparation of crystalline Form-K Febuxostat, i.e. 2-(3-cyano- 4-isobutyloxyphenyl)-4-methyl-5-thiazolecarboxylic acid.

### Instrumentation

### Powder X-ray Diffraction (PXRD)

The said polymorph of the present invention is characterized by their X-ray powder diffraction pattern. Thus, the X-ray diffraction patterns of said polymorphs of the invention were measured on 1) ***PANalytical, X'Pert PRO*** powder diffractometer equipped with goniometer of Ø/Ø configuration and ***X'Celerator*** detector and 2) ***Bruker AXS D8 Discover*** powder X-ray diffractometer equipped with a goniometer of 0/20 configuration, Vario 1 monochromator and ***Lynx-Eye*** detector. The Cu-anode X-ray tube was operated at 40kV and 30mA. The experiments were conducted over the 2Ø range of 2.0°-50.0°, 0.030° step size and 50 seconds step time.

### Differential Scanning Calorimetry (DSC)

The DSC measurements were carried out on TA Q1000 of TA instruments. The experiments were performed at a heating rate of 10.0 °C/min over a temperature range of 30 - 300 °C purging with nitrogen at a flow rate of 50 mL/min. Standard aluminum crucibles covered by lids with three pin holes were used.

### Thermo gravimetric Analysis (TGA)

TGA/DTA was recorded using the instrument TA Q5000 of TA instruments. The experiments were performed at a heating rate of 10.0 °C/min over a temperature range of 30 - 300 °C purging with nitrogen at a flow rate of 25 mL/min.

Oneaspect of the present disclosure is to provide crystalline Form-M₁ of Febuxostat.

In one aspect of this disclosure, crystalline Febuxostat Form M₁ is characterized by the powder X- ray diffraction having characteristic peaks 6.13, 9.06, 12.29, 17.44 and 25.81±0.2° 2Ø.

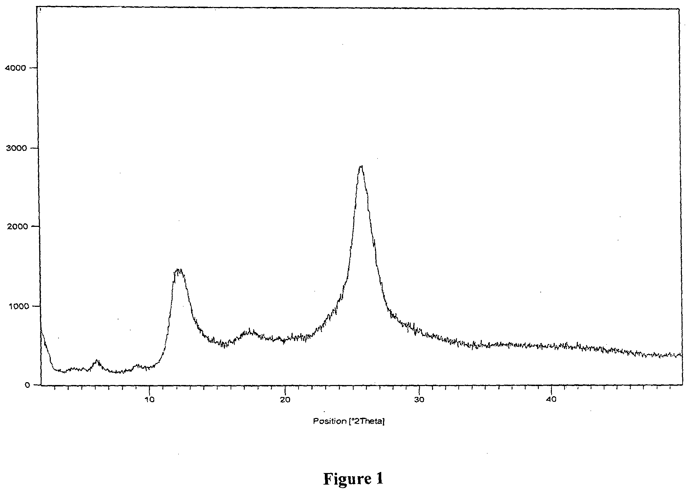

In another aspect of this disclosure crystalline Form-M₁ of Febuxostat is further characterized by the Powder X-ray diffraction as depicted in Figure 1.

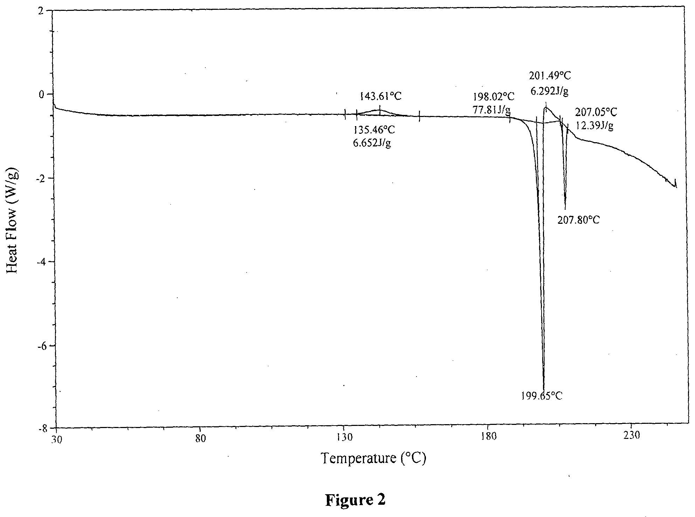

In one more aspect of this disclosure , crystalline Form-M₁ of Febuxostat is further characterized by the DSC thermogram as depicted in Figure 2.
Another aspect of the present disclosure provides a process for the preparation of Febuxostat crystalline Form-M₁ comprising the steps of:
a) dissolving Febuxostat in an ester solvent,
b) spray drying the clear solution, and
c) isolating the crystalline Form-M₁.

The ester solvent used herein may be Ethyl acetate.

The input Febuxostat used herein is selected from the group consisting of but not limited to crystalline or amorphous form or any solvate.

Febuxostat crystal is dissolved in ester solvent such as ethyl acetate at 50 - 60 °C and cooled to room temperature. The obtained solution optionally filtered to remove any undissolved particulate, and the clear solution is subjected to spray drying in a buchi mini spray dryer (Model B-290) to obtain crystalline Febuxostat Form-M₁ .

The present invention provides an improved process for the preparation of crystalline Form-K of Febuxostat comprising the steps of:
a) dissolving Febuxostat in an ester solvent at 50 to 80°C ,
b) evaporating the solvent by agitated thin film dryer (ATFD), and
c) isolating Febuxostat crystalline Form-K.
In one embodiment, the ester solvent used herein is Ethyl acetate.

In another embodiment, the input Febuxostat used herein is selected from the group consisting of but not limited to crystalline or amorphous form or any solvate.

According to present invention Febuxostat is dissolved in ester solvent such as ethyl acetate at 50 - 60 °C and cooled to room temperature. The obtained solution optionally filtered to remove any undissolved particulate, and the clear solution is evaporated on agitated thin film dryer (ATFD) instrument, at 50 °C under reduced pressure (120 mm-Hg) to obtain Febuxostat Form-K.

Another aspect of the present invention is to provide an improved process for the preparation of crystalline Form-K of Febuxostat comprising the steps of:
a) dissolving Febuxostat in an ester solvent at 50 - 80 °C,
b) removing the solvent at same temperature, and
c) isolating Febuxostat crystalline Form-K.

In one embodiment, the ester solvent used herein is Ethyl acetate.

In one more embodiment, the solvent removed in step b, is at temperature 70 - 80 °C.
a) In one more embodiment, the input Febuxostat used herein is selected from the group consisting of but not limited to crystalline or amorphous form or any solvate.

According to present invention Febuxostat is dissolved in ester solvent such as ethyl acetate at 50 - 60 °C, cooled to room temperature (25 - 30 °C). The resulting solution is filtered through hyflow to remove any undissolved particulate and the solution was heated to 70 - 80 °C. The solvent was distilled out at the same temperature under reduced pressure to obtain Febuxostat Form-K.

Yet another aspect of the present invention is to provide an improved process for the preparation of crystalline Form-K of Febuxostat comprising the steps of:
a) dissolving Febuxostat in an ester solvent at 50 - 80 °C,
b) removing the solvent,
c) adding hydrocarbon solvent, and
d) isolating Febuxostat crystalline Form-K.

In one embodiment, the ester solvent used herein is Ethyl acetate.

In one more embodiment, the solvent removed in step b, is at temperature 70 - 80 °C.

In another embodiment, hydrocarbon solvents used herein are selected from cyclohexane and toluene.

In one more embodiment, the input Febuxostat used herein is selected from the group consisting of but not limited to crystalline or amorphous form or any solvate.

According to present invention Febuxostat is dissolved in ester solvent such as ethyl acetate at 50 - 60 °C, cooled to room temperature (25 - 30 °C). The resulting solution is filtered through hyflow to remove any undissolved particulate and the solution was heated to 70 - 80 °C. The solvent is distilled out at the same temperature under reduced pressure and hydrocarbon solvents such as toluene or cyclohexane is added to the residue. The resulting is slurred, filtered and dried to obtain Febuxostat Form-K .

Yet another aspect of the present invention is to provide an improved process for the preparation of crystalline Form-K of Febuxostat comprising the steps of:
a) dissolving Febuxostat in an ester solvent at 50 - 80 °C,
b) filtering the reaction mass,
c) maintaining the filtrate for 30-120 minutes at same temperature,
d) removing the solvent,
e) adding hydrocarbon solvent, and
f) isolating Febuxostat crystalline Form-K.

In one embodiment, the ester solvent used herein is Ethyl acetate.

In one more embodiment, the reaction mass is filtered to separate any undissolved particulate.

In one more embodiment, the filtrate is maintained for 30-120 minutes, preferably 60-90 minutes at 50 - 80 °C.

In one more embodiment, at step d, the solvent is removed at a temperature of 30 - 70 °C under reduced pressure.

In another embodiment, hydrocarbon solvent used herein is selected from cyclohexane and toluene.

In one more embodiment, the input Febuxostat used herein is selected from the group consisting of but not limited to crystalline or amorphous form or any solvate.

Yet another aspect of the present invention is to provide an improved process for the preparation of crystalline Form-K of Febuxostat comprising the steps of:
a) dissolving Febuxostat in one or more organic solvents,
b) adding a base to the reaction mixture,
c) removing the solvent,
d) adding water and neutralizing with acid,
e) adding one or more ester solvents,
f) optionally adding hydrocarbon solvent, and
g) isolating Febuxostat crystalline Form-K.

In one embodiment, the organic solvent is selected from ethanol, methanol, isopropanol, tetrahydrofuran or mixtures thereof.

In one more embodiment, base is selected from alkali or alkali earth metal hydroxides like sodium hydroxide or potassium hydroxide, preferably sodium hydroxide.

In one more embodiment, acid used in this invention for the neutralization is selected from acetic acid, formic acid, hydrochloric acid, sulfuric acid or phosphoric acid.

In another embodiment, the ester solvent used herein is Ethyl acetate.

In another embodiment, hydrocarbon solvents used herein are selected from cyclohexane or toluene.

In one more embodiment, the input Febuxostat used herein is selected from the group consisting of but not limited to crystalline or amorphous form or any solvate.

According to the present invention Febuxostat is dissolved in organic solvents such as ethanol and tetrahydrofuran at 25 - 30 °C. To this base such as sodium hydroxide is added and the solvent is distilled out at 60 - 70 °C. The reaction mass is cooled to 25 - 30 °C and water is added. This is neutralized with hydrochloric acid and ester solvent like ethyl acetate is added. The reaction mass is completely distilled out at 75 - 80 °C under reduced pressure and cooled to 25 - 30 °C. To the solid mass cyclohexane is added and filtered.

The resulting solid is dried to obtain Febuxostat crystalline Form-K.

Yet another aspect of the present invention is to provide an improved process for the preparation of crystalline Form-K of Febuxostat comprising the steps of:
a) dissolving alkyl ester of Febuxostat in one or more organic solvents,
b) adding a base to the reaction mixture,
c) removing the solvent,
d) adding water and neutralizing with acid,
e) adding one or more ester solvents,
f) optionally adding hydrocarbon solvent, and
g) isolating Febuxostat crystalline Form-K.

In one embodiment, alkyl ester of Febuxostat is selected from methyl or ethyl ester, preferably ethyl ester.
In one more embodiment, the organic solvent is selected from ethanol, methanol, isopropanol, tetrahydrofuran or mixtures thereof.
In one more embodiment, base is selected from alkali or alkali earth metal hydroxides like sodium hydroxide or potassium hydroxide, preferably sodium hydroxide.

In one more embodiment, acid used in this invention for the neutralization is selected from acetic acid, formic acid, hydrochloric acid, sulfuric acid and phosphoric acid.

In another embodiment, the ester solvent used herein is Ethyl acetate.

In another embodiment, hydrocarbon solvents used herein are selected from cyclohexane and toluene.

According to the present invention ethyl ester of Febuxostat is dissolved in organic solvents such as ethanol and tetrahydrofuran at 25 - 30 °C. To this base such as sodium hydroxide is added and the solvent is distilled out at 60 - 70 °C. The reaction mass is cooled to 25 - 30 °C and water is added. This is neutralized with hydrochloric acid and ester solvent like ethyl acetate is added. The reaction mass is completely distilled out at 75 - 80 °C under reduced pressure and cooled to 25 - 30 °C. To the solid mass cyclohexane is added and filtered. The resulting solid is dried to obtain Febuxostat crystalline Form-K.

Yet another aspect of the present invention is to provide an improved process for the preparation of crystalline Form-K of Febuxostat comprising the steps of:
a) dissolving Febuxostat in an ester solvent,
b) partially removing the solvent,
c) adding hydrocarbon solvent, and
d) isolating Febuxostat crystalline Form-K.

In one embodiment, the ester solvent used herein is Ethyl acetate.

In another embodiment, hydrocarbon solvents used herein are selected from cyclohexane and toluene.
In one more embodiment, the input Febuxostat used herein is selected from the group consisting of but not limited to crystalline or amorphous form or any solvate.

Yet another aspect of the present invention is to provide an improved process for the preparation of crystalline Form-K of Febuxostat comprising the steps of:
a) dissolving Febuxostat in mixture of ester and hydrocarbon solvent,
b) removing the solvent, and
c) isolating Febuxostat crystalline Form-K.

In one embodiment, the ester solvent used herein is Ethyl acetate and hydrocarbon solvent used herein is selected from cyclohexane and toluene. The preferable mixture of solvent is mixture of ethyl acetate and toluene.

In one more embodiment, at step b, 30-70 % preferably 40-55 % of solvent is removed at a temperature of 50 - 100 °C preferably at atmospheric pressure.

In one more embodiment, Febuxostat crystalline Form K is isolated by cooling the reaction temperature to 10 -40 °C, preferably 20- 35 °C followed by filtration.

In one more embodiment, the input Febuxostat used herein is selected from the group consisting of but not limited to crystalline or amorphous form or any solvate.

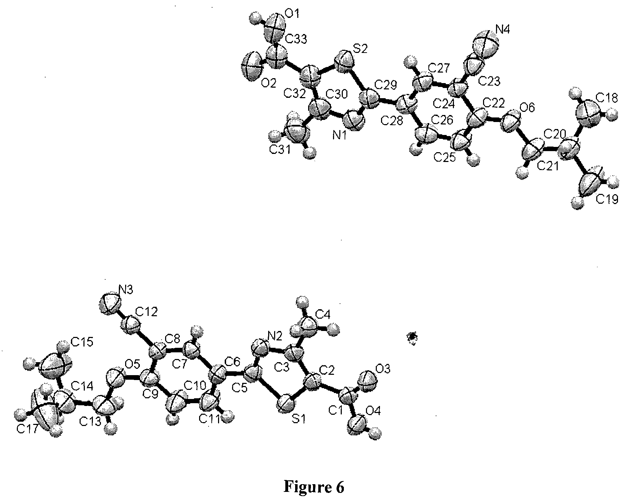

Febuxostat Form K is characterized by single crystal X-ray diffraction as shown in Figure 6. The crystallographic data and atomic coordinates are incorporated respectively in Table 1 and Table 2.

**Table 1: Crystallographic Data of Febuxostat Form K from single crystal X-ray diffraction.**

| | |
|---|---|
| Crystal system | Triclinic |
| Lattice type | Primitive |
| Space group | *P*-1 |
| Lattice parameters | *a* = 7.3660(15)Å |
| | *b* = 15.403(3)Å |
| | *c* = 15.721(3)Å |
| | *α* = 90.85(3)° |
| | *β* = 96.38(3)° |
| | *γ* = 98.75(3)° |
| | *V* = 1751.1(6) Å³ |
| Reflections (I > 2 σ) | 4048 |
| R-values: R, Rw | 0.0777, 0.1626 |

**Table 2: Atomic coordinates of Febuxostat Form K obtained from single crystal X-ray diffraction.**

| | Coordinates | | | |
|---|---|---|---|---|
| Atom | x | y | z | Ueq |
| S1 | 0.37859(14) | 0.06001(6) | 0.76398(6) | 0.0599 |
| S2 | 0.11124(14) | 0.87630(7) | 0.25622(7) | 0.0672 |
| O1 | 0.0449(5) | 0.9901(2) | 0.3948(2) | 0.0961 |
| O2 | 0.0308(5) | 0.8924(2) | 0.4972(2) | 0.0995 |
| O3 | 0.4619(4) | 0.1019(2) | 0.52581(18) | 0.0779 |
| O4 | 0.4517(5) | -0.0160(2) | 0.60628(19) | 0.0804 |
| O5 | 0.2721(4) | 0.20255(17) | 1.15913(17) | 0.0769 |
| O6 | 0.2427(4) | 0.65396(16) | -0.09539(19) | 0.0729 |
| N1 | 0.1411(5) | 0.7189(2) | 0.2993(2) | 0.0708 |
| N2 | 0.3579(4) | 0.2249(2) | 0.76064(19) | 0.0549 |
| N3 | 0.2567(6) | 0.4085(2) | 1.0843(3) | 0.0868 |
| N4 | 0.2208(7) | 0.8748(3) | - 0.1236(3) | 0.1091 |
| C1 | 0.4420(5) | 0.0666(3) | 0.5940(3) | 0.0588 |
| C2 | 0.4022(5) | 0.1134(2) | 0.6696(2) | 0.0537 |
| C3 | 0.3871(5) | 0.2005(3) | 0.6801(2) | 0.0554 |
| C4 | 0.3986(6) | 0.2695(3) | 0.6138(3) | 0.0728 |
| C5 | 0.3504(5) | 0.1573(2) | 0.8113(2) | 0.0549 |
| C6 | 0.3215(5) | 0.1651(2) | 0.9021(2) | 0.0539 |
| C7 | 0.3092(5) | 0.2466(2) | 0.9363(2) | 0.052 |
| C8 | 0.2890(5) | 0.2566(2) | 1.0228(2) | 0.0519 |
| C9 | 0.2837(6) | 0.1845(2) | 1.0765(2) | 0.0602 |
| C10 | 0.2909(7) | 0.1027(3) | 1.0403(3) | 0.0818 |
| C11 | 0.3104(6) | 0.0934(3) | 0.9550(3) | 0.0749 |
| C12 | 0.2721(6) | 0.3410(3) | 1.0582(2) | 0.0606 |
| C13 | 0.2752(7) | 0.1332(3) | 1.2197(3) | 0.0868 |
| C14 | 0.2871(8) | 0.1747(4) | 1.3081(3) | 0.0975 |
| C15 | 0.1266(12) | 0.2194(5) | 1.3184(4) | 0.1854 |
| C17 | 0.4701(12) | 0.2329(5) | 1.3304(4) | 0.1826 |
| C18 | 0.1187(10) | 0.5774(4) | - 0.2664(4) | 0.1467 |
| C19 | 0.3108(10) | 0.4601(4) | - 0.2265(4) | 0.1361 |
| C20 | 0.2784(9) | 0.5544(3) | - 0.2078(4) | 0.0916 |
| C21 | 0.2471(7) | 0.5621(3) | - 0.1152(3) | 0.0797 |
| C22 | 0.2208(5) | 0.6774(2) | -0.0149(3) | 0.0618 |
| C23 | 0.2120(7) | 0.8251(3) | -0.0708(3) | 0.0735 |
| C24 | 0.2042(5) | 0.7658(2) | - 0.0014(3) | 0.0572 |
| C25 | 0.2138(6) | 0.6218(3) | 0.0537(3) | 0.0692 |
| C26 | 0.1907(6) | 0.6537(3) | 0.1328(3) | 0.0692 |
| C27 | 0.1792(5) | 0.7959(3) | 0.0797(3) | 0.0617 |
| C28 | 0.1718(5) | 0.7409(3) | 0.1480(3) | 0.059 |
| C29 | 0.1449(5) | 0.7708(3) | 0.2339(3) | 0.0613 |
| C30 | 0.1120(6) | 0.7622(3) | 0.3716(3) | 0.0748 |
| C31 | 0.1088(7) | 0.7132(4) | 0.4537(3) | 0.1013 |
| C32 | 0.0904(5) | 0.8488(3) | 0.3604(3) | 0.0699 |
| C33 | 0.0547(6) | 0.9132(4) | 0.4225(3) | 0.0736 |
| H1 | 0.023(8) | 1.0218(11) | 0.4336(11) | 0.1443 |
| H4 | 0.493(7) | -0.0369(9) | 0.5654(16) | 0.1208 |
| H4a | 0.386(4) | 0.2419(3) | 0.5579(3) | 0.1092 |
| H4b | 0.301(2) | 0.3039(12) | 0.6172(12) | 0.1092 |
| H4c | 0.5160(17) | 0.3070(12) | 0.6239(11) | 0.1092 |
| H7 | 0.3144(5) | 0.2950(2) | 0.9016(2) | 0.0624 |
| H10 | 0.2826(7) | 0.0536(3) | 1.0742(3) | 0.0978 |
| H11 | 0.3162(6) | 0.0380(3) | 0.9322(3) | 0.0897 |
| H13a | 0.166(3) | 0.0890(15) | 1.2083(4) | 0.104 |
| H13b | 0.379(3) | 0.1022(19) | 1.2145(5) | 0.104 |
| H14 | 0.2827(8) | 0.1267(4) | 1.3485(3) | 0.117 |
| H15a | 0.137(5) | 0.243(4) | 1.3759(15) | 0.278 |
| H15b | 0.124(6) | 0.266(3) | 1.279(4) | 0.278 |
| H15c | 0.0147(13) | 0.1780(13) | 1.307(5) | 0.278 |
| H17a | 0.468(4) | 0.2881(18) | 1.303(4) | 0.274 |
| H17b | 0.493(5) | 0.243(4) | 1.3913(6) | 0.274 |
| H17c | 0.5666(17) | 0.205(2) | 1.311(4) | 0.274 |
| H18a | 0.0075(16) | 0.540(2) | -0.256(2) | 0.22 |
| H18b | 0.105(5) | 0.6374(12) | -0.256(2) | 0.22 |
| H18c | 0.141(4) | 0.570(3) | -0.3249(4) | 0.22 |
| H19a | 0.411(5) | 0.4463(13) | -0.187(2) | 0.204 |
| H19b | 0.200(2) | 0.4198(5) | -0.220(3) | 0.204 |
| H19c | 0.341(7) | 0.4552(10) | - 0.2839(12) | 0.204 |
| H20 | 0.394(8) | 0.599(4) | - 0.226(4) | 0.15 |
| H21a | 0.347(3) | 0.5419(15) | -0.0791(7) | 0.096 |
| H21b | 0.131(2) | 0.5269(11) | -0.1059(14) | 0.096 |
| H25 | 0.2248(6) | 0.5629(3) | 0.0460(3) | 0.0827 |
| H26 | 0.1874(6) | 0.6157(3) | 0.1782(3) | 0.0831 |
| H27 | 0.1673(5) | 0.8545(3) | 0.0880(3) | 0.0739 |
| H31a | -0.0126(16) | 0.708(2) | 0.4718(13) | 0.151 |
| H31b | 0.140(5) | 0.6557(9) | 0.4445(7) | 0.151 |
| H31c | 0.197(4) | 0.7448(13) | 0.4971(7) | 0.151 |

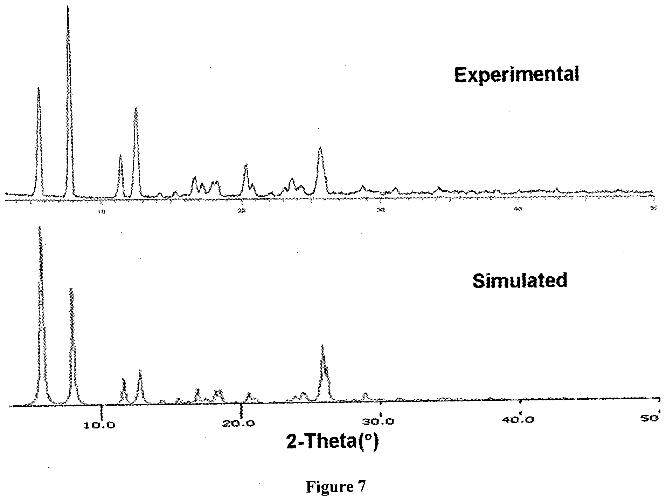

The experimental PXRD pattern was matched with the simulated PXRD pattern obtained from single crystal X-ray diffraction as shown in Figure 7, which shows the phase purity of Form K.

A further aspect of the present invention is to provide packaging conditions for the stable crystalline Form-K of Eebuxostat in a way to attain the polymorphic stability, there by increasing the shelf life of the product. According to the present invention the method for packaging crystalline Febuxostat Form-K comprises of placing Febuxostat Form-K in LDPE (low density polyethylene) bag under nitrogen atmosphere, placing the sealed bag in Triple laminated aluminum liner bag with vacuumised nitrogen sealing, placing the above Triple laminated aluminum bag into the outer bag of triple laminated aluminum bag and vacuumised nitrogen sealing and enclosing the triple laminated bag in closed HDPE (high density polyethylene) drums.

The invention is illustrated with the following examples, which are provided by way of illustration only and should not be construed to limit the scope of the invention.

### Experimental procedure:

### Comparative Example - 1: Process for the Preparation of crystalline Febuxostat Form-M₁

5 g of Febuxostat Crystal A was dissolved in ethyl acetate (200 mL) at 50 - 60 °C and cooled to room temperature (25 - 30 °C). The resulting solution was filtered through hyflow to remove any undissolved particulate. The clear solution was subjected to spray drying in a Buchi mini spray dryer (Model B-290). The product was collected and dried at 50 °C under vacuum for 15h. The resulted solid was identified as crystalline Febuxostat Form -M₁.

### Comparative Example - 2: Process for the Preparation of crystalline Febuxostat Form-M₁

5 g of Febuxostat Crystal G was dissolved in ethyl acetate (200 mL) at 50 - 60 °C and cooled to room temperature (25 - 30 °C). The resulting solution was filtered through hyflow to remove any undissolved particulate. The clear solution was subjected to spray drying in a Buchi Mini Spray Dryer (B-290). The product was collected and dried at 50 °C under vacuum for 15h. The resulted solid was identified as crystalline febuxostat Form-M₁.

### Comparative Example - 3: Process for the preparation of Febuxostat crystal Form-K

5 g of Febuxostat Crystal A was dissolved in ethyl acetate (200 mL) at 50 - 60 °C and cooled to room temperature (25 - 30 °C). The resulting solution was filtered through hyflow to remove any undissolved particulate. The clear solution was evaporated on agitated thin film dryer (ATFD) instrument, at 50 °C under reduced pressure (120 mm-Hg). The resulted solid was collected and identified as crystalline febuxostat Form K.

### Comparative Example - 4: Process for the preparation of Febuxostat crystal Form-K

5g of Febuxostat Crystal G was dissolved in ethyl acetate (200 mL) at 50 - 60 °C and cooled to room temperature (25 - 30 °C). The resulting solution was filtered through hyflow to remove any undissolved particulate. The clear solution was evaporated on ATFD instrument at 65 °C under reduced pressure (120 mm-Hg). The resulted solid was collected and identified as crystalline febuxostat Form K.

### Example - 5: Process for the preparation of Febuxostat crystal Form-K

5 g of Febuxostat was dissolved in ethyl acetate (200 mL) at 70 - 80 °C and the solvent was removed on rotary evaporator at same temperature under reduced pressure. The resulted solid was collected and identified as crystalline febuxostat Form K.

### Example - 6: Process for the preparation of Febuxostat crystal Form-K

10 g of Febuxostat was dissolved in ethyl acetate (150 mL) at 70 - 80 °C and the solvent was removed on rotary evaporator at same temperature under reduced pressure. The resulted solid was collected and identified as crystalline febuxostat Form K.

### Comparative Example - 7: Process for the preparation of Febuxostat crystal Form-K

5 g of Febuxostat was dissolved in ethyl acetate (200 mL) at 50 - 60 °C and cooled to room temperature (25 - 30 °C). The resulting solution was filtered through hyflow to remove any undissolved particulate. The solution was heated to 70 - 80 °C and the solvent was distilled out at the same temperature under reduced pressure. The resulted solid was collected and identified as crystalline febuxostat Form K.

### Example - 8: Process for the preparation of Febuxostat crystal Form-K

10 g of Febuxostat was dissolved in ethyl acetate (250 mL) at 50 - 60 °C and cooled to room temperature (25 - 30 °C). The resulting solution was filtered through hyflow to remove any undissolved particulate. The solution was heated to 70 - 80 °C and the solvent was distilled out at the same temperature under reduced pressure. To the resulted solid mass 100 mL of toluene was added, slurred for 2 hours, filtered and dried over night at 50 °C under reduced pressure. The dry solid was identified as crystalline febuxostat Form K.

### Example - 9: Process for the preparation of Febuxostat crystal Form-K

10 g of Febuxostat was dissolved in ethyl acetate (250 mL) at 50 - 60 °C, cooled to room temperature (25 - 30 °C). The resulting solution was filtered through hyflow to remove any undissolved particulate. The solution was heated to 70 - 80 °C and the solvent was distilled out at the same temperature under reduced pressure. To the resulted solid mass 100 mL of cyclohexane was added, slurred for 2 hours, filtered and dried over night at 50 °C under reduced pressure. The dry solid was identified as crystalline febuxostat Form K.

### Example - 10: Process for the preparation of Febuxostat crystal Form-K

10 g of Febuxostat was dissolved in ethyl acetate (250 mL) at 50 - 80 °C. The resulting solution was filtered and maintained for 60-90 minutes at 50 - 80 °C. The solvent was distilled out at 40-60 °C under reduced pressure. To the resulted solid mass 100 mL of cyclohexane was added and maintained for 2 hours. The reaction mass is centrifuged and the wet material is dried at 60 °C under reduced pressure. The dry solid was identified as crystalline Febuxostat Form.

### Comparative Example - 11: Process for the preparation of Febuxostat crystal Form-K

5 g of Febuxostat was suspended in ethyl acetate (80 mL) at 25-30°C and heated to 75 - 80 °C to obtain clear solution. The clear solution was distilled at 75 - 80 °C under reduced pressure to remove 60 mL of solvent. To the resulting suspension, cyclohexane (50 mL) was added at 75 - 80 °C and stirred for 10 - 15 minutes at 75 - 80 °C. The reaction mass was further distilled to half of its volume at 70 - 80 °C under reduced pressure and filtered at 75 - 80 °C. The product was isolated and dried at 60 °C under vacuum for 12 - 15 h. The solid obtained was identified as crystalline Febuxostat Form K.

### Comparative Example - 12: Process for the preparation of Febuxostat crystal Form-K

20 g of Febuxostat was suspended in EtOH (160 mL) and THF (220 mL) at 25 - 30 °C and stirred for 10 - 15 min to obtain clear solution. To this clear solution, aqueous NaOH solution (IN, 108 mL) was added slowly at 25 - 30 °C, raised the temperature to 60 °C and stirred the reaction mass at 60 °C for 1 h. The solvent was distilled out completely under reduced pressure at 60-70 °C. The reaction mass was cooled to 25 - 30 °C, added water (100 mL) and neutralized with IN HC1. Then ethyl acetate (400 mL) was added to the reaction mass and stirred for 10 - 15 minutes at 25-30°C. The aqueous layer was extracted using ethyl acetate (100 mL) and dried over anhydrous Na₂SO₄. The reaction mass was distilled out completely at 75 - 80 °C under reduced pressure. The solid obtained was identified as crystalline Febuxostat Form K.

### Example - 13: Process for the preparation of Febuxostat crystal Form-K

20g of Febuxostat was suspended in EtOH (160 mL) and THF (220 mL) at 25-30 °C and stirred for 10 - 15 min to obtain clear solution. To this clear solution, aqueous NaOH solution (IN, 108 mL) was added slowly at 25 - 30°C, raised the temperature to 60 °C and stirred the reaction mass at 60 °C for lh. The solvent was distilled out completely under reduced pressure at 60 - 70 °C. The reaction mass was cooled to 25 - 30 °C, added water (100 mL) and neutralized with IN HC1. Then ethyl acetate (400 mL) was added to the reaction mass and stirred for 10 - 15 minutes at 25-30°C. The aqueous layer was extracted using ethyl acetate (100 mL) and dried over anhydrous Na₂SO₄. The reaction mass was distilled out completely at 75 - 80 °C under reduced pressure and cooled to 25 - 30°C. To the resulting solid mass, cyclohexane (100 mL) was added, slurred for 2 hours at 25-30°C and filtered. The isolated solid was dried at 60°C under vacuum for 12 - 15h. The product obtained was identified as crystalline Febuxostat Form K.

### Example - 14: Process for the preparation of Febuxostat crystal Form-K

20g of ethyl ester of Febuxostat [ethyl 2-(3-cyano-4-isobutyloxyphenyl)-4-methyl-5-thiazolecarboxylate] was suspended in EtOH (160 mL) and of THF (220 mL) at 25-30°C and stirred for 10 - 15 min to obtain clear solution. To this clear solution, aqueous NaOH solution (IN, 108 mL) was added slowly at 25 - 30°C, raised the temperature to 60°C and stirred the reaction mass at 60°C for lh. The solvent was distilled out completely under reduced pressure at 60 - 70°C. The reaction mass was cooled to 25 - 30°C, added water (100 mL) and neutralized with IN HC1. Then ethyl acetate (400 mL) was added to the reaction mass and stirred for 10 - 15 minutes at 25 - 30°C. The aqueous layer was extracted using ethyl acetate (100 mL) and dried over anhydrous Na₂SO₄. The reaction mass was distilled out completely at 75 - 80 °C under reduced pressure and cooled to 25 - 30 °C. To the resulting solid mass, cyclohexane (100 mL) was added, slurred for 2 hours at 25 - 30 °C and filtered. The isolated solid was dried at 60 °C under vacuum for 12 - 15 h. The product obtained was identified as crystalline Febuxostat Form K.

### Comparative Example - 15: Process for the preparation of Febuxostat crystal Form-K

50 g of Febuxostat was suspended in ethyl acetate (1250 mL) at 25 - 30 °C and heated to 75 - 80 °C to obtain clear solution. The resulted solution was refluxed for lh and then cooled to 60 °C. The solvent was distilled at 60 - 35 °C under reduced pressure to leave about 400 - 500 mL of solvent. To the resulting suspension, cyclohexane (500 mL) was added at 35 °C and stirred for lh at 25 - 30 °C and filtered. The product was isolated, dried at 60 °C under vacuum and unloaded under controlled humidity 40± 10 % RH and packed. The solid obtained was identified as crystalline Febuxostat Form K.

### Comparative Example - 16: Process for the preparation of Febuxostat crystal Form-K

50 g of Febuxostat was suspended in ethyl acetate (1000 mL) and toluene (500 mL) at 25 - 30 °C and heated to 75 - 80 °C to obtain clear solution. The resulted solution was refluxed for lh. The solution was distilled at 80 - 90 °C to leave about 700 mL of solution. The clear solution was cooled to 70 °C in 30 minutes and maintain at 70°C for further 30 minutes. The hazy solution was further cooled to 25 - 30 °C in about lh and maintain at 25 - 30 °C for 30 minutes. The slurry was filtered, dried at 60 °C under vacuum and unloaded under controlled humidity 40± 10 % RH and packed. The solid obtained was identified as crystalline Febuxostat Form K.

### Packaging:

Febuxostat Form-K was packed in LDPE bag under nitrogen atmosphere, twisted and tied with plastic fastener. It was inserted in Triple laminated aluminum liner bag with vacuumised nitrogen sealing. Both these bags were then put into the outer bag of triple laminated aluminum bag and vacuumised nitrogen sealing. Such poly bags were further packed in HDPE drums, closed with plastic lids having rubber gasket followed by locking ring and a metal seal and labeled.

## Claims

1. An improved process for the preparation of crystalline Form-K of Febuxostat comprising the steps of:
a) dissolving Febuxostat in an ester solvent at 50 - 80 °C,
b) filtering the reaction mass,
c) maintaining the filtrate for 30-120 minutes at same temperature,
d) removing the solvent,
e) adding hydrocarbon solvent, and
f) isolating Febuxostat crystalline Form-K.

2. The process according to claim 1, wherein:
(i) the ester solvent is ethyl acetate; and/or
(ii) the hydrocarbon solvent is selected from cyclohexane or toluene.

3. An improved process for the preparation of crystalline Form-K of Febuxostat comprising the steps of:
a) dissolving Febuxostat in an ester solvent at 50 - 80 °C,
b) removing the solvent and
c) isolating Febuxostat crystalline Form-K.

4. The process according to claim 3 , wherein the ester solvent is ethyl acetate.

5. An improved process for the preparation of crystalline Form-K of Febuxostat comprising the steps of:
a) dissolving Febuxostat or an ethyl ester of Febuxostat in one or more organic solvents,
b) adding a base to the reaction mixture,
c) removing the solvent by distillation at 60-70°C,
d) cooling the reaction mass to 25-30°C, adding water and neutralizing with hydrochloric acid,
e) adding one or more ester solvents,
f) removing the ester solvent by distillation at 75-80°C under reduced pressure,
g) cooling the reaction mass to 25-30°C,
h) adding cyclohexane, and
i) isolating Febuxostat crystalline Form-K.

6. The process according to claim 5, wherein:
(i) the organic solvent is selected from ethanol, methanol, isopropanol, tetrahydrofuran or mixtures thereof; and/or
(ii) the base is selected from alkali or alkali earth metal hydroxides; and/or
(iii) the ester solvent is ethyl acetate.

## Patentansprüche

1. Verbessertes Verfahren zur Herstellung der kristallinen Form K von Febuxostat, umfassend die Schritte des:
a) Auflösens von Febuxostat in einem Esterlösungsmittel bei 50 - 80 °C,
b) Filterns der Reaktionsmasse,
c) Haltens des Filtrats für 30-120 Minuten auf derselben Temperatur,
d) Entfernens des Lösungsmittels,
e) Zugebens von Kohlenwasserstoff-Lösungsmittel und des
f) Isolierens von Febuxostat in der kristallinen Form K.

2. Verfahren nach Anspruch 1, wobei:
(i) das Esterlösungsmittel Ethylacetat ist und/oder
(ii) das Kohlenwasserstoff-Lösungsmittel aus Cyclohexan oder Toluol ausgewählt ist.

3. Verbessertes Verfahren zur Herstellung der kristallinen Form K von Febuxostat, umfassend die Schritte des:
a) Auflösens von Febuxostat in einem Esterlösungsmittel bei 50 - 80 °C,
b) Entfernens des Lösungsmittels und des
c) Isolierens von Febuxostat in der kristallinen Form K.

4. Verfahren nach Anspruch 3, wobei das Esterlösungsmittel Ethylacetat ist.

5. Verbessertes Verfahren zur Herstellung der kristallinen Form K von Febuxostat, umfassend die Schritte des:
a) Auflösens von Febuxostat oder eines Ethylesters von Febuxostat in einem oder mehreren organischen Lösungsmitteln,
b) Zugebens einer Base zum Reaktionsgemisch,
c) Entfernens des Lösungsmittels durch Destillation bei 60-70 °C,
d) Abkühlens der Reaktionsmasse auf 25-30 °C, des Zugebens von Wasser und des Neutralisierens mit Salzsäure,
e) Zugebens eines oder mehrerer Esterlösungsmittel,
f) Entfernens des Esterlösungsmittels durch Destillation bei 75-80 °C unter reduziertem Druck,
g) Abkühlens der Reaktionsmasse auf 25-30°C,
h) Zugebens von Cyclohexan und des
i) Isolierens von Febuxostat in der kristallinen Form K.

6. Verfahren nach Anspruch 5, wobei:
(i) das organische Lösungsmittel aus Ethanol, Methanol, Isopropanol, Tetrahydrofuran oder Gemischen davon ausgewählt ist und/oder
(ii) die Base aus Alkali- oder Erdalkalimetallhydroxiden ausgewählt ist und/oder
(iii) das Esterlösungsmittel Ethylacetat ist.

## Revendications

1. Processus amélioré pour la préparation d'une forme K cristalline du fébuxostat, comprenant les étapes consistant à :
a) dissoudre le fébuxostat dans un solvant ester à 50 à 80 °C,
b) filtrer la masse de réactionnelle,
c) maintenir le filtrat pendant 30 à 120 minutes à la même température,
d) éliminer le solvant,
e) ajouter un solvant hydrocarbure, et
f) isoler la forme K cristalline du fébuxostat.

2. Processus selon la revendication 1, dans lequel :
(i) le solvant ester est l'acétate d'éthyle ; et/ou
(ii) le solvant hydrocarbure est choisi parmi le cyclohexane ou le toluène.

3. Processus amélioré pour la préparation d'une forme K cristalline du fébuxostat comprenant les étapes consistant à :
a) dissoudre le fébuxostat dans un solvant ester à 50 à 80 °C,
b) éliminer le solvant et
c) isoler le forme K cristalline de fébuxostat.

4. Processus selon la revendication 3, dans lequel le solvant ester est l'acétate d'éthyle.

5. Processus amélioré pour la préparation d'une forme K cristalline du fébuxostat, comprenant les étapes consistant à :
a) dissoudre le fébuxostat ou un ester d'éthyle de fébuxostat dans au moins un solvant organique,
b) ajouter une base au mélange réactionnel,
c) éliminer le solvant par distillation à 60 à 70 °C,
d) refroidir la masse réactionnelle jusqu'à 25 à 30 °C, ajouter de l'eau et neutraliser avec de l'acide chlorhydrique,
e) ajouter au moins un solvant ester,
f) éliminer le solvant ester par distillation à 75 à 80 °C sous pression réduite,
g) refroidir la masse réactionnelle jusqu'à 25 à 30 °C,
h) ajouter du cyclohexane, et
i) isoler la forme K cristalline du fébuxostat.

6. Processus selon la revendication 5, dans lequel :
(i) le solvant organique est choisi parmi l'éthanol, le méthanol, l'isopropanol, le tétrahydrofurane ou leurs mélanges ; et/ou
(ii) la base est choisie parmi des hydroxydes de métaux alcalins ou alcalino-terreux ; et/ou
(iii) le solvant ester est l'acétate d'éthyle.
